# EUROPEAN PATENT SPECIFICATION

(11) **EP 2 154 251 B1**
(45) Date of publication and mention of the grant of the patent: **04.09.2013**
(21) Application number: 08748503.3
(22) Date of filing: 15.05.2008
(51) Int. Cl.: C12N 15/63, C07K 14/00, C07K 14/475, C07K 14/005, A61K 38/16, A61K 38/18, A61K 39/12, A61P 31/12, C12P 21/02, C07K 19/00, C12N 15/62

(54) **EXPRESSION SYSTEM OF RECOMBINANT PROTEINS WITH ENHANCED YIELD AND IMMUNOGENICITY**
EXPRESSIONSSYSTEM FÜR REKOMBINANTE PROTEINE MIT VERBESSERTER AUSBEUTE UND IMMUNOGENITÄT
SYSTÈME D'EXPRESSION DE PROTÉINE RECOMBINANTE À RENDEMENT ET IMMUNOGÉNICITÉ AMÉLIORÉS

(30) Priority: 15.05.2007 CN 200710107917
(43) Date of publication of application: 17.02.2010
(73) Proprietor: Animal Technology Institute Taiwan, Chunan Miaoli Taiwan (CN)
(72) Inventor: CHEN, Mingyu, Miaoli County 350 Taiwan (CN); CHUANG, Chinkai, Hsinchu City 300 Taiwan (CN); SU, Yuhsyu, Miaoli County 351 Taiwan (CN); FAN, Chiuting, Hsinchu County 302 Taiwan (CN); YU, Jungchuan, Hsinchu County 302 Taiwan (CN); LEE, Wen-Chuan, Hsinchu City, Taiwan (CN)
(74) Representative: Viering, Jentschura & Partner
(86) International application number: PCT/CN2008/000948
(87) International publication number: WO 2008/138229

(56) References cited:
- WO-A2-02/055684
- CN-A- 1 189 103
- CN-A- 1 283 702
- CN-A- 1 491 116
- US-A1- 2002 086 844
- KIM ET AL: "TAT-Hsp40 inhibits oxidative stress-mediated cytotoxicity via the inhibition of Hsp70 ubiquitination" FEBS LETTERS, ELSEVIER, AMSTERDAM, NL LNKD- DOI:10.1016/J.FEBSLET.2008.01.053, vol. 582, no. 5, 5 February 2008 (2008-02-05), pages 734-740, XP022495814 ISSN: 0014-5793
- LAI Y ET AL: "Selectively increasing inducible heat shock protein 70 via TAT-protein transduction protects neurons from nitrosative stress and excitoxicity" JOURNAL OF NEUROCHEMISTRY, WILEY INTERSCIENCE, NEW YORK, NY, US LNKD- DOI:10.1111/J.1471-4159.2005.03212.X, vol. 94, no. 2, 1 July 2005 (2005-07-01), pages 360-366, XP008103300 ISSN: 0022-3042 [retrieved on 2005-05-23]
- BEERENS A M J ET AL: "Protein transduction domains and their utility in gene therapy" CURRENT GENE THERAPY, BENTHAM SCIENCE PUBLISHERS LTD, NL, vol. 3, no. 5, 1 October 2003 (2003-10-01) , pages 486-494, XP008090797 ISSN: 1566-5232
- REIMANN JOERG ET AL: "DNA vaccines expressing antigens with a stress protein-capturing domain display enhanced immunogenicity" IMMUNOLOGICAL REVIEWS, vol. 199, no. 1, June 2004 (2004-06), pages 54-67, XP002589225 ISSN: 0105-2896
- X-B QIU ET AL: "The diversity of the DnaJ/Hsp40 family, the crucial partners for Hsp70 chaperones" CMLS CELLULAR AND MOLECULAR LIFE SCIENCES, BIRKHÄUSER-VERLAG, BA LNKD- DOI:10.1007/S00018-006-6192-6, vol. 63, no. 22, 4 September 2006 (2006-09-04), pages 2560-2570, XP019457689 ISSN: 1420-9071
- CHUANG C K ET AL: "A dual-functional E. coli vector for expressing recombinant protein with high solubility and antigen presentation ability" PROTEIN EXPRESSION AND PURIFICATION, ACADEMIC PRESS, SAN DIEGO, CA LNKD- DOI:10.1016/J.PEP.2008.12.011, vol. 65, no. 1, 1 May 2009 (2009-05-01), pages 51-56, XP025959405 ISSN: 1046-5928 [retrieved on 2008-12-30]
- REIMANN J. ET AL.: 'DNA vaccines expressing antigens with a stress protein-capturing domain display enhanced immunogenicity' IMMUNOLOGICAL REVIEWS vol. 199, no. 1, 2004, pages 54 - 67, XP002589225
- LAI Y. ET AL.: 'Selectively increasing inducible heat shock protein 70 via TAT-protein transduction protects neurons from nitrosative stress and excitotoxicity' J. NEUROCHEM. vol. 94, no. 2, 2005, pages 360 - 366, XP008103300
- QIU X.B. ET AL.: 'The diversity of the DnaJ/Hsp40 family, the crucial partners for Hsp70 chaperones' CELL. MOL. LIFE SCI. vol. 63, no. 22, 04 September 2006, pages 2560 - 2570, XP019457689

### SEQUENCE LISTING

<110> Animal Technology Institute Taiwan
<120> EXPRESSION SYSTEM FOR ENHANCING SOLUBILITY AND IMMUNOGENEICITY OF
   RECOMBINANT PROTEINS
<130> 100014-00002
<160> 68
<170> PatentIn version 3.3
<210> 1
   <211> 11
   <212> PRT
   <213> Human immunodeficiency virus
<220>
   <223> Human immunodeficiency virus Tat PTD1
<400> 1
   Tyr Gly Arg Lys Lys Arg Arg Gln Arg Arg Arg
   1               5                   10
<210> 2
   <211> 33
   <212> DNA
   <213> Artificial sequenece
<220>
   <223> Human immunodeficiency virus Tat PTD1
<400> 2
   tatggtcgta agaaacgtcg tcagcgtcgt cgt 33
<210> 3
   <211> 72
   <212> PRT
   <213> Homo sapiens
<220>
   <223> HSP40 J-domain (J1)
<400> 3
   Gly Lys Asp Tyr Tyr Gln Thr Leu Gly Leu Ala Arg Gly Ala Ser Asp
   1               5                   10                  15
   Asp Glu Ile Lys Arg Ala Tyr Arg Arg Gln Ala Leu Arg Tyr His Pro
               20                  25                  30
   Asp Lys Asn Lys Glu Pro Arg Ala Glu Glu Lys Phe Lys Glu Ile Ala
           35                  40                  45
   Tyr Asp Val Leu Ser Asp Pro Arg Lys Arg Lys Arg Glu Ile Phe Asp
       50                  55                  60
   Arg Tyr Gly Glu Glu Gly Leu Lys
   65                  70
<210> 4
   <211> 216
   <212> DNA
   <213> Artificial Sequenece
<220>
   <223> HSP40 J-domain (J1)-encoding sequence
<400> 4
   ggtaaagatt actaccagac tcacggtctc gctcgtggtg catctgatga tgaaatcaaa      60
   cgtgcttacc gtcgtcaggc actgcgttac catccagaca aaaacaaaga accgcgtgca     120
   gaagagaaat tcaaagagat cgcagaagca tacgacgttc tgagcgatcc acgtaaacgt     180
   gaaatctccg accgttacgg tgaagaaggt ctgaaa                               216
<210> 5
   <211> 39
   <212> DNA
   <213> Artificial sequenece
<220>
   <223> Forward primer PTD1 f for synthesis of PTD1
<400> 5
   tatgtatggt cgtaagaaac gtcgtcagcg tcgtcgtgg 39
<210> 6
   <211> 41
   <212> DNA
   <213> Artificial Sequenece
<220>
   <223> Forward primer PTD1 r for synthesis of PTD1
<400> 6
   gatcccacga cgacgctgac gacgtttctt acgaccatac a 41
<210> 7
   <211> 39
   <212> DNA
   <213> Artificial Sequenece
<220>
   <223> Forward primer J1 nBF1 for synthesis of HSP40 J-domain
<400> 7
   tggatcctgg gtaaagatta ctaccagact cacggtctc 39
<210> 8
   <211> 48
   <212> DNA
   <213> Artificial Sequenece
<220>
   <223> Forward primer nF2 for synthesis of HSP40 J-domain
<400> 8
   tactaccaga ctcacggtct cgctcgtggt gcatctgatg atgaaatc 48
<210> 9
   <211> 48
   <212> DNA
   <213> Artificial Sequenece
<220>
   <223> Forward primer F3 for synthesis of HSP40 J-domain
<400> 9
   ggtgcatctg atgatgaaat caaacgtgct taccgtcgtc aggcactg 48
<210> 10
   <211> 48
   <212> DNA
   <213> Artificial Sequenece
<220>
   <223> Forward primer F4 for synthesis of HSP40 J-domain
<400> 10
   gcttaccgtc gtcaggcact gcgttaccat ccagacaaaa acaaagaa 48
<210> 11
   <211> 45
   <212> DNA
   <213> Artificial sequenece
<220>
   <223> Forward primer F5 for synthesis of HSP40 J-domain
<400> 11
   taccatccag acaaaaacaa agaaccgggt gcagaagaga aattc 45
<210> 12
   <211> 48
   <212> DNA
   <213> Artificial Sequenece
<220>
   <223> Forward primer F6 for synthesis of HSP40 J-domain
<400> 12
   ccgggtgcag aagagaaatt caaagagatc gcagaagcat acgacgtt 48
<210> 13
   <211> 36
   <212> DNA
   <213> Artificial Sequenece
<220>
   <223> Reverse primer EcoR1 R1 for synthesis of HSP40 J-domain
<400> 13
   cgaattcgca ccaccagaac cacctttcag accttc 36
<210> 14
   <211> 48
   <212> DNA
   <213> Artificial Sequenece
<220>
   <223> Reverse primer R2 for synthesis of HSP40 J-domain
<400> 14
   agaaccacct ttcagacctt cttcaccgta acggtcgaag atttcacg 48
<210> 15
   <211> 48
   <212> DNA
   <213> Artificial sequenece
<220>
   <223> Reverse primer R3 for synthesis of HSP40 J-domain
<400> 15
   gtaacggtcg aagatttcac gtttacgtgg atcgctcaga acgtcgta 48
<210> 16
   <211> 36
   <212> DNA
   <213> Artificial Sequenece
<220>
   <223> Reverse primer R4 for synthesis of HSP40 J-domain
<400> 16
   tggatcgctc agaacgtcgt atgcttctgc gatctc 36
<210> 17
   <211> 171
   <212> DNA
   <213> Artificial Sequenece
<220>
   <223> Human HSPA1A peptide I-encoding sequence
<400> 17
   agcagcagca cccaggcgag cctggaaatt gatagcctgt ttgaaggcat tgatttttat 60
   accagcatta cccgtgcgcg ttttgaagaa ctgtgcagcg atctgtttcg tagcaccctg 120
   gaaccggtgg aaaaagcgct gcgtgatgcg aaactggata aagcgcagat t 171
<210> 18
   <211> 57
   <212> PRT
   <213> Homo sapiens
<220>
   <223> Human HSPA1A peptide I
<400> 18
   Ser Ser Ser Thr Gln Ala Ser Leu Glu Ile Asp Ser Leu Phe Glu Gly
   1 5 10 15
   Ile Asp Phe Tyr Thr Ser Ile Thr Arg Ala Arg Phe Glu Glu Leu Cys
   20 25 30
   Ser Asp Leu Phe Arg Ser Thr Leu Glu Pro Val Glu Lys Ala Leu Arg
   35 40 45
   Asp Ala Lys Leu Asp Lys Ala Gln Ile
   50 55
<210> 19
   <211> 49
   <212> DNA
   <213> Artificial Sequenece
<220>
   <223> Forward primer RI-I-f1 for synthesis of HSPA1A peptide I
<400> 19
   gaattctagc agcagcaccc aggcgagcct ggaaattgat agcctgttt 49
<210> 20
   <211> 48
   <212> DNA
   <213> Artificial Sequenece
<220>
   <223> Forward primer I-f2 for synthesis of HSPA1A peptide I
<400> 20
   agcctggaaa ttgatagcct gtttgaaggc attgattttt ataccagc 48
<210> 21
   <211> 49
   <212> DNA
   <213> Artificial Sequenece
<220>
   <223> Forward primer I-f3 for synthesis of HSPA1A peptide I
<400> 21
   gtttgaaggc attgattttt ataccagcat tacccgtgcg cgttttgaa 49
<210> 22
   <211> 49
   <212> DNA
   <213> Artificial Sequenece
<220>
   <223> Reverse primer I-r3 for synthesis of HSPA1A peptide I
<400> 22
   gggtgctacg aaacagatcg ctgcacagtt cttcaaaacg cgcacgggt 49
<210> 23
   <211> 49
   <212> DNA
   <213> Artificial Sequenece
<220>
   <223> Reverse primer I-r2 for synthesis of HSPA1A peptide I
<400> 23
   catcacgcag cgctttttcc accggttcca gggtgctacg aaacagatc 49
<210> 24
   <211> 48
   <212> DNA
   <213> Artificial Sequenece
<220>
   <223> Reverse primer xho-I-r1 for synthesis of HSPA1A peptide I
<400> 24
   ctcgagaatc tgcgctttat ccagtttcgc atcacgcagc gctttttc 48
<210> 25
   <211> 183
   <212> DNA
   <213> Artificial Sequenece
<220>
   <223> Human HSPA1A peptide II-encoding sequence
<400> 25
   aacgtaaccg ctactgacaa atccactggt aaagctaaca agatcaccat caccaacgac 60
   aaaggtcgtc tgtccaagga agagatcgag cgtatggttc aggaagctga aaagtacaag 120
   gctgaagacg aagttcagcg tgaacgtgtt tccgctaaga acgctctgga atcctacgct 180
   ttc 183
<210> 26
   <211> 61
   <212> PRT
   <213> Homo sapiens
<220>
   <223> Human HSPA1A peptide II
<400> 26
   Asn Val Thr Ala Thr Asp Lys Ser Thr Gly Lys Ala Asn Lys Ile Thr
   1 5 10 15
   Ile Thr Asn Asp Lys Gly Arg Leu Ser Lys Glu Glu Ile Glu Arg Met
   20 25 30
   Val Gln Glu Ala Glu Lys Tyr Lys Ala Glu Asp Glu Val Gln Arg Glu
   35 40 45
   Arg Val Ser Ala Lys Asn Ala Leu Glu Ser Tyr Ala Phe
   50 55 60
<210> 27
   <211> 49
   <212> DNA
   <213> Artificial Sequenece
<220>
   <223> Forward primer RI-II-f1 for synthesis of HSPA1A peptide II
<400> 27
   gaattctaac gtaaccgcta ctgacaaatc cactggtaaa gctaacaag 49
<210> 28
   <211> 49
   <212> DNA
   <213> Artificial Sequenece
<220>
   <223> Forward primer II-f2 for synthesis of HSPA1A peptide II
<400> 28
   tccactggta aagctaacaa gatcaccatc accaacgaca aaggtcgtc 49
<210> 29
   <211> 49
   <212> DNA
   <213> Artificial Sequenece
<220>
   <223> Forward primer II-f3 for synthesis of HSPA1A peptide II
<400> 29
   atcaccaacg acaaaggtcg tctgtccaag gaagagatcg agcgtatgg 49
<210> 30
   <211> 45
   <212> DNA
   <213> Artificial Sequenece
<220>
   <223> Forward primer II-f4 for synthesis of HSPA1A peptide II
<400> 30
   aaggaagaga tcgagcgtat ggttcaggaa gctgaaaagt acaag 45
<210> 31
   <211> 46
   <212> DNA
   <213> Artificial Sequenece
<220>
   <223> Reverse primer II-r3 for synthesis of HSPA1A peptide II
<400> 31
   acgctgaact tcgtcttcag ccttgtactt ttcagcttcc tgaacc 46
<210> 32
   <211> 45
   <212> DNA
   <213> Artificial Sequenece
<220>
   <223> Reverse primer II-r2 for synthesis of HSPA1A peptide II
<400> 32
   agcgttctta gcggaaacac gttcacgctg aacttcgtct tcagc 45
<210> 33
   <211> 49
   <212> DNA
   <213> Artificial Sequenece
<220>
   <223> Reverse primer xho-II-r1 for synthesis of HSPA1A peptide II
<400> 33
   ctcgaggaaa gcgtaggatt ccagagcgtt cttagcggaa acacgttca 49
<210> 34
   <211> 183
   <212> DNA
   <213> Artificial Sequenece
<220>
   <223> Human HSPA1A peptide III-encoding sequence
<400> 34
   gaagatgaag gcctgaaagg caaaattagc gaagcggata agaaaaaggt gctggataaa 60
   tgccaggaag tgattagctg gctggatgcg aacaccctgg cggaaaaaga tgaatttgaa 120
   cataaacgta aagaactgga acaggtgtgc aacccgatta ttagcggcct gtatcagggc 180
   gcg 183
<210> 35
   <211> 61
   <212> PRT
   <213> Homo sapiens
<220>
   <223> Human HSPA1A peptide III
<400> 35
   Glu Asp Glu Gly Leu Lys Gly Lys Ile Ser Glu Ala Asp Lys Lys Lys
   1 5 10 15
   Val Leu Asp Lys Cys Gln Glu Val Ile Ser Trp Leu Asp Ala Asn Thr
   20 25 30
   Leu Ala Glu Lys Asp Glu Phe Glu His Lys Arg Lys Glu Leu Glu Gln
   35 40 45
   Val Cys Asn Pro Ile Ile Ser Gly Leu Tyr Gln Gly Ala
   50 55 60
<210> 36
   <211> 46
   <212> DNA
   <213> Artificial Sequenece
<220>
   <223> Forward primer RI-III-f1 for synthesis of HSPA1A peptide III
<400> 36
   gaattctgaa gatgaaggcc tgaaaggcaa aattagcgaa gcggat 46
<210> 37
   <211> 48
   <212> DNA
   <213> Artificial Sequenece
<220>
   <223> Forward primer III-f2 for synthesis of HSPA1A peptide III
<400> 37
   ggcaaaatta gcgaagcgga taagaaaaag gtgctggata aatgccag 48
<210> 38
   <211> 49
   <212> DNA
   <213> Artificial Sequenece
<220>
   <223> Forward primer III-f3 for synthesis of HSPA1A peptide III
<400> 38
   ggtgctggat aaatgccagg aagtgattag ctggctggat gcgaacacc 49
<210> 39
   <211> 49
   <212> DNA
   <213> Artificial Sequenece
<220>
   <223> Forward primer III-f4 for synthesis of HSPA1A peptide III
<400> 39
   gctggatgcg aacaccctgg cggaaaaaga tgaatttgaa cataaacgt 49
<210> 40
   <211> 45
   <212> DNA
   <213> Artificial Sequenece
<220>
   <223> Reverse primer III-r3 for synthesis of HSPA1A peptide III
<400> 40
   ttccagttct ttacgtttat gttcaaattc atctttttcc gccag 45
<210> 41
   <211> 48
   <212> DNA
   <213> Artificial Sequenece
<220>
   <223> Reverse primer III-r2 for synthesis of HSPA1A peptide III
<400> 41
   cgggttgcac acctgttcca gttctttacg tttatgttca aattcatc 48
<210> 42
   <211> 48
   <212> DNA
   <213> Artificial Sequenece
<220>
   <223> Reverse primer III-r1 for synthesis of HSPA1A peptide III
<400> 42
   ctcgagcgcg ccctgataca ggccgctaat aatcgggttg cacacctg 48
<210> 43
   <211> 210
   <212> DNA
   <213> Artificial Sequenece
<220>
   <223> chicke IGF-1 encoding sequence
<400> 43
   ggtccagaaa ccctgtgtgg tgcagaactg gttgatgcac tgcagttcgt gtgtggtgat 60
   cgtggtttct acttcagcaa accgactggt tatggtagct ctagccgtcg tctgcatcac 120
   aaaggtattg tggatgaatg ttgctttcag agctgtgatc tgcgtcgtct ggaaatgtac 180
   tgtgcaccaa tcaaaccacc gaaaagcgca 210
<210> 44
   <211> 70
   <212> PRT
   <213> Gallus gallus
<220>
   <223> chicke IGF-1
<400> 44
   Gly Pro Glu Thr Leu Cys Gly Ala Glu Leu Val Asp Ala Leu Gln Phe
   1 5 10 15
   Val Cys Gly Asp Arg Gly Phe Tyr Phe Ser Lys Pro Thr Gly Tyr Gly
   20 25 30
   Ser Ser Ser Ala Ala Leu His His Lys Gly Ile Val Asp Glu Cys Cys
   35 40 45
   Phe Gln Ser Cys Asp Leu Arg Arg Leu Glu Met Tyr Cys Ala Pro Ile
   50 55 60
   Lys Pro Pro Lys Ser Ala
   65 70
<210> 45
   <211> 58
   <212> DNA
   <213> Artificial Sequenece
<220>
   <223> Forward primer RI-F1 new for synthesis of cIGF-I
<400> 45
   gaattctggt ccagaaaccc tgtgtggtgc agaactggtt gatgcactgc agttcgtg 58
<210> 46
   <211> 48
   <212> DNA
   <213> Artificial Sequenece
<220>
   <223> Forward primer F2 for synthesis of cIGF-I
<400> 46
   gaactggttg atgcactgca gttcgtgtgt ggtgatcgtg gtttctac 48
<210> 47
   <211> 51
   <212> DNA
   <213> Artificial Sequenece
<220>
   <223> Forward primer F3 for synthesis of cIGF-I
<400> 47
   ggtgatcgtg gtttctactt cagcaaaccg actggttatg gtagctctag c 51
<210> 48
   <211> 49
   <212> DNA
   <213> Forward primer F4 for synthesis of cIGF-I
<220>
   <223> oligonucleotides
<400> 48
   ggttatggta gctctagccg tcgtctgcat cacaaaggta ttgtggatg 49
<210> 49
   <211> 50
   <212> DNA
   <213> Artificial sequenece
<220>
   <223> Forward primer F5 for synthesis of cIGF-I
<400> 49
   cacaaaggta ttgtggatga atgttgcttt cagagctgtg atctgcgtcg 50
<210> 50
   <211> 45
   <212> DNA
   <213> Artificial Sequenece
<220>
   <223> Reverse primer R2 for synthesis of cIGF-I
<400> 50
   gattggtgca cagtacattt ccagacgacg cagatcacag ctctg 45
<210> 51
   <211> 46
   <212> DNA
   <213> Artificial Sequenece
<220>
   <223> Reverse primer xho-R1 for synthesis of cIGF-I
<400> 51
   ctcgagtgcg cttttcggtg gtttgattgg tgcacagtac atttcc 46
<210> 52
   <211> 489
   <212> DNA
   <213> Artificial Sequenece
<220>
   <223> Avian Influenza HA (H5) RBD-encoding sequence
<400> 52
   aaacacctat tgagcagaat aaaccatttt gagaaaattc agatcatccc caaaagttct 60
   tggtccagtc atgaagcctc attaggggtg agctcagcat gtccatacca gggaaagtcc 120
   tcctttttca gaaatgtggt atggcttatc aaaaagaaca gtacataccc aacaataaag 180
   aggagctaca ataataccaa ccaagaagat cttttggtac tgtgggggat tcaccatcct 240
   aatgatgcgg cagagcagac aaagctctat caaaacccaa ccacctatat ttccgttggg 300
   acatcaacac taaaccagag attggtacca agaatagcta ctagatccga agtaaacggg 360
   caaagtggaa ggatggagtt cttctggaca attttaaaac cgaatgatgc aatcaacttc 420
   gagagtaatg gaaatttcat tgctccagaa tatgcataca aaattgtcaa gaaaggggac 480
   tcaacaatt 489
<210> 53
   <211> 163
   <212> PRT
   <213> Avian Influenza virus HA subtype H5
<220>
   <223> Avian Influenza HA (H5) RBD
<400> 53
   Lys His Leu Leu Ser Arg Ile Asn His Phe Glu Lys Ile Gln Ile Ile
   1 5 10 15
   Pro Lys Ser Ser Trp Ser Ser His Glu Ala Ser Leu Gly Val Ser Ser
   20 25 30
   Ala Cys Pro Tyr Gln Gly Lys Ser Ser Phe Phe Arg Asn Val Val Trp
   35 40 45
   Leu Ile Lys Lys Asn Ser Thr Tyr Pro Thr Ile Lys Arg Ser Tyr Asn
   50 55 60
   Asn Thr Asn Gln Glu Asp Leu Leu Val Leu Trp Gly Ile His His Pro
   65 70 75 80
   Asn Asp Ala Ala Glu Gln Thr Lys Leu Tyr Gln Asn Pro Thr Thr Tyr
   85 90 95
   Ile Ser Val Gly Thr Ser Thr Leu Asn Gln Arg Leu Val Pro Arg Ile
   100 105 110
   Ala Thr Arg Ser Glu Val Asn Gly Gln Ser Gly Arg Met Glu Phe Phe
   115 120 125
   Trp Thr Ile Leu Lys Pro Asn Asp Ala Ile Asn Phe Glu Ser Asn Gly
   130 135 140
   Asn Phe Ile Ala Pro Glu Tyr Ala Tyr Lys Ile Val Lys Lys Gly Asp
   145 150 155 160
   Ser Thr Ile
<210> 54
   <211> 32
   <212> DNA
   <213> Artificial Sequenece
<220>
   <223> Forward primer RI HA-RBD f for synthesis of avian influenza HA RBD
<400> 54
   tgaattcgaa acacctattg agcagaataa ac 32
<210> 55
   <211> 31
   <212> DNA
   <213> Artificial Sequenece
<220>
   <223> Reverse primer Xho HA-RBD r for synthesis of avian influenza HA RBD
<400> 55
   tctcgagaat tgttgagtcc cctttcttga c 31
<210> 56
   <211> 573
   <212> DNA
   <213> Artificial Sequenece
<220>
   <223> Hepatitis C virus core protein-encoding sequence
<400> 56
   atgagcacaa atcctaaacc tcaaagaaaa accaaaagaa acaccaaccg tcgcccagaa     60
   gacgttaagt tcccgggcgg cggccagatc gttggcggag tatacttgtt gccgcgcagg    120
   ggccccaggt tgggtgtgcg cacgacaagg aaaacttcgg agcggtccca gccacgtggg    180
   agacgccagc ccatccccaa agatcggcgc tccactggca aggcctgggg aaaaccaggt    240
   cgcccctggc ccctatatgg gaatgaggga ctcggctggg caggatggct cctgtccccc    300
   cgaggctctc gcccctcctg gggccccact gacccccggc ataggtcgcg caacgtgggt    360
   aaagtcatcg acaccctaac gtgtggcttt gccgacctca tggggtacat ccccgtcgta    420
   ggcgccccgc ttagtggcgc cgccagagct gtcgcgcacg gcgtgagagt cctggaggac    480
   ggggttaatt atgcaacagg gaacctaccc ggtttcccct tttctatctt cttgctggcc    540
   ctgttgtcct gcatcaccgt tccggtctct gct                                 573
<210> 57
   <211> 191
   <212> PRT
   <213> Hepatitis C virus
<220>
   <223> Hepatitis C virus core protein
<400> 57
   Met Ser Thr Asn Pro Lys Pro Gln Arg Lys Thr Lys Arg Asn Thr Asn
   1               5                   10                  15
   Arg Arg Pro Glu Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Thr
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ala Trp Gly Lys Pro Gly
   65                  70                  75                  80
   Arg Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg His Arg Ser Arg Asn Val Gly Lys Val Ile Asp Thr Leu Thr Cys
   115 120 125
   Gly Phe Ala Asp Leu Met Gly Tyr Ile Pro Val Val Gly Ala Pro Leu
   130 135 140
   Ser Gly Ala Ala Arg Ala Val Ala His Gly Val Arg Val Leu Leu Asp
   145 150 155 160
   Gly Val Asn Tyr Ala Thr Gly Asn Leu Pro Gly Phe Pro Phe Ser Ile
   165 170 175
   Phe Leu Leu Ala Leu Leu Ser Cys Ile Thr Val Pro Val Ser Ala
   180 185 190
<210> 58
   <211> 30
   <212> DNA
   <213> Artificial Sequenece
<220>
   <223> Forward primer RI-Core f for HCV-core protein
<400> 58
   tgaattcgat gagcacaaat cctaaacctc 30
<210> 59
   <211> 28
   <212> DNA
   <213> Artificial Sequenece
<220>
   <223> Reverse primer xho-Core r for for HCV-core protein
<400> 59
   tctcgagagc agagaccgga acggtgat 28
<210> 60
   <211> 105
   <212> DNA
   <213> Artifical Sequence
<220>
   <223> Fusion gene encoding Human Haptoglobin Fusion Polypeptide HpNC
<400> 60
   gatagcggta acgatgtgac cgatattgcg gatgatggtg gtagcggtgg tcgtcattat 60
   gaaggtagca ccgttccgga gaagaaaacc ccaaagagcg gtggt 105
<210> 61
   <211> 35
   <212> PRT
   <213> Homo Sapiens
<220>
   <223> Human Haptoglobin Fusion Polypeptide HpNC
<400> 61
   Asp Ser Gly Asn Asp Val Thr Asp Ile Ala Asp Asp Gly Gly Ser Gly
   1               5                   10                  15
   Gly Arg His Tyr Glu Gly Ser Thr Val Pro Glu Lys Lys Thr Pro Lys
               20                  25                  30
   Ser Gly Gly
           35
<210> 62
   <211> 49
   <212> DNA
   <213> Artificial Sequenece
<220>
   <223> Forward primer RI-F1 for Human Haptoglobin fusion polypeptide HpNC
<400> 62
   agaattctga tagcggcaac gatgtgaccg atattgcgga tgatggtgg 49
<210> 63
   <211> 44
   <212> DNA
   <213> Artificial Sequenece
<220>
   <223> Forward primer F2 for Human Haptoglobin fusion polypeptide HpNC
<400> 63
   tgaccgatat tgcggatgat ggtggtagtg gtggtcgtca ttat 44
<210> 64
   <211> 41
   <212> DNA
   <213> Artificial Sequenece
<220>
   <223> Forward primer F3 for Human Haptoglobin fusion polypeptide HpNC
<400> 64
   atggtggtag tggtggtcgt cattatgaag gtagcaccgt t 41
<210> 65
   <211> 47
   <212> DNA
   <213> Artificial Sequenece
<220>
   <223> Reverse primer R2 for Human Haptoglobin fusion polypeptide HpNC
<400> 65
   ggttttcttc tccggaacgg tgctaccttc ataatgacga ccaccac 47
<210> 66
   <211> 47
   <212> DNA
   <213> Artificial Sequenece
<220>
   <223> Reverse primer xho-R1 for Human Haptoglobin fusion polypeptide HpNC
<400> 66
   tctcgagacc accgctcttt ggggttttct tctccggaac ggtgcta 47
<210> 67
   <211> 99
   <212> DNA
   <213> Oryctolagus cuniculus
<220>
   <223> cDNA encoding rabbit Neutrophile peptide-1 (NP-1)
<400> 67
   gtggtctgtg cgtgcagacg agccctctgt ttgcctcggg aacgtcgtgc tgggttctgc 60
   agaatccgtg gaagaatcca cccactctgc tgccgccgc 99
<210> 68
   <211> 33
   <212> PRT
   <213> Oryctolagus cuniculus
<220>
   <223> rabbit Neutrophile peptide-1 (NP-1)
<400> 68
   Val Val Cys Ala Cys Arg Arg Ala Leu Cys Leu Pro Arg Glu Arg Arg
   1 5 10 15
   Ala Gly Phe Cys Arg Ile Arg Gly Arg Ile His Pro Leu Cys Cys Arg
   20 25 30
   Arg

## Description

### BACKGROUND OF THE INVENTION

The present invention relates to an expression system of recombinant proteins with an enhanced yield and immunogenicity.

A demand for efficient production of biologics for therapeutic use has steadily increased as more products, such as recombinant proteins, are approved or are nearing approval for use in humans. Bacterial fermentation processes have long been, and still are, the major tool for production of these types of molecules. The key objective of process optimization is to attain a high yield of product having the required quality at the lowest possible cost, which is often determined by the properties of a specific expression construct or system.

It is reported that the structural features of J-domains determine the specificity of interaction between Hsp40 proteins and their partner Hsp70s [Hennessy et al., Protein Science (2005), 14:1697-1709]. J-domain is believed to be the major binding site and the minimal region required for interaction between Hsp40 and Hsp40-like protein with their partner Hsp70s [Walsh et al., EMBO report (2004), 6: 567-571]. It has been reported that a peptide fused to the J-domain of SV40 large T antigen by a DNA vaccine system could enhance the antigenicity of the peptide [Reimann and Schirmbeck, Immuno Rev. (2004), 199:54-67]. It is also known that the J-domain tethers DnaK to DnaJ-bound substrates, which DnaK then binds with its C-terminal peptide-binding domain [Tilstra et al., Biochemical Society Transactions (2007), 35:811-815].

On the other hand, protein transduction domains (PTDs) are small peptides that are able to ferry much larger molecules into cells independent of classical endocytosis. This property makes PTDs ideal tools to transfer proteins and other molecules into living cells for research purposes [Beerens et al., Current Gene Therapy (2003), 3(5):486-494].

US 2002/0086844 A1 discloses chimeric proteins for modulating apoptosis in a cell, comprising at least one J-domain preferably from DnaJ/Hsp40 and the PTD of HIV-1 TAT. WO 02/055684 A2 and Beerens A M J et al. (Current Gene Therapy, vol. 3, no.5, pages 486-494) teach that PTD improves the bioavailability of the expressed protein or transfers a protein and another molecule into living cells. And in LAI Y et al (Journal of Neurochemistry, vol. 94, no.2, pages 360-366), PTD is reported to have the ability of protein transduction so as to translocate TAT-Hsp70 fusion protein into the neural cells.

However, neither the PTD nor Hsp40 J-domain has been implied in the development of the expression system that improves yield and immunogenicity of the recombinant protein.

### BRIEF SUMMARY OF THE INVENTION

In accordance with the invention, there is provided a method for expressing a recombinant target protein with an enhanced yield in a host cell. The method of the invention comprises:
constructing an expression vector comprising a nucleic acid segment consisting of a first nucleotide sequence encoding a protein transduction domain (PTD) of SEQ ID NO: 1, a second nucleotide sequence, linked in translation frame with the first nucleotide sequence, encoding a Hsp40 J-domain of SEQ ID NO:3, and a third nucleotide sequence, linked in translation frame with the second nucleotide sequence, encoding a target protein, wherein the nucleic acid segment is operatively linked to host cell specific transcription and translation regulatory elements;
transforming the expression vector in the host cell; and
collecting and isolating the recombinant target protein from the host cell.

Therefore, the invention also provides the recombinant protein produced by using the above mentioned method. Accordingly, the immunogenicity of the recombinant protein as resulted is enhanced.

The present invention may also provide a vaccine comprising the above-described recombinant protein.

The present invention also provides a chimeric protein comprising:
a. a first polypeptidyl fragment at the N-terminal end of the chimeric protein that consists of a protein transduction domain (PTD) of SEQ ID NO: 1;
b. a second polypeptidyl fragment at the C-terminal end of the first polypeptidyl fragment that consists of a Hsp40 J-domain of SEQ ID NO:3; and
c. a third polypeptidyl fragment at the C-terminal end of the second polypeptidyl fragment that contains a target protein or polypeptide.

The present invention provides a chimeric gene comprising:
a. a first DNA sequence encoding a protein transduction domain (PTD) consisting of an amino acid sequence of SEQ ID NO:1; and
b. a second DNA sequence, linked in translation frame with the first DNA sequence, encoding a Hsp40 J-domain consisting of an amino acid sequence of SEQ ID NO:3.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### BRIEF DESCRIPTION OF THE SEVERAL VIEWS OF THE DRAWINGS

For the purpose of illustrating the invention, there are shown in the drawings embodiments which are presently preferred. It should be understood, however, that the invention is not limited to the embodiments shown.

In the drawings:

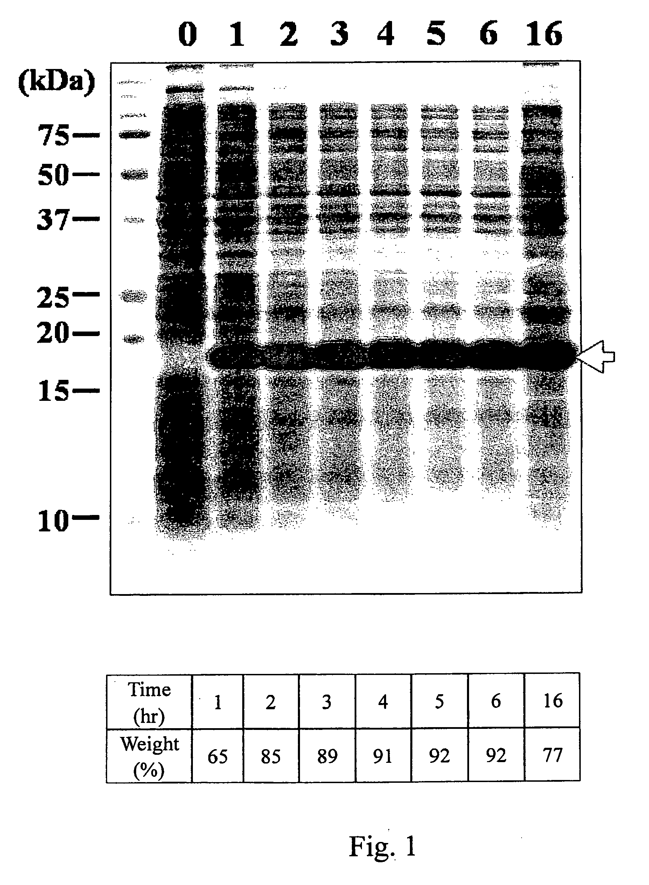

Fig. 1 shows a Coomassie® blue stained SDS-PAGE of whole cell lysates of *E*. *coli* Rosetta containing the pET22b-PTD-J-HSPA1A peptide I constructs grown in the presence of IPTG. First lane, standard molecular weight marker (bands corresponding to molecular mass of 15 kDa and 20 kDa are shown). Second lane (0), uninduced pET22b-PTD-J-HSPAIA peptide I; Third lane (1), induced pET22b-PTD-J-HSPA1A peptide I after 1 hour; Fourth lane (2), induced pET22b-PTD-J-HSPA1A peptide I after 2 hours; Fifth lane (3), induced pET22b-PTD-J-HSPA1A peptide I after 3 hours; Sixth lane (4), induced pET22b-PTD-J-HSPAIA peptide I after 4 hours; Seventh lane (5), induced pET22b-PTD-J-HSPAIA peptide I after 5 hours; Eighth lane (6), induced pET22b-PTD-J-HSPA1A peptide I after 6 hours; and Ninth lane (16), induced pET22b-PTD-J-HSPAIA peptide I after 16 hours.

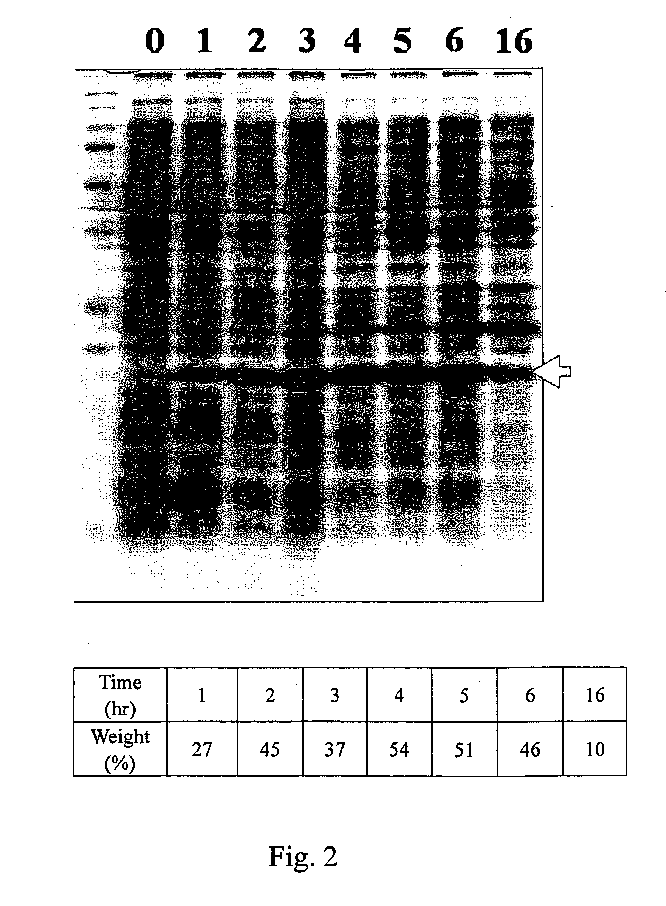

Fig. 2 shows a Coomassie® blue stained SDS-PAGE of whole cell lysates of *E*. *coli* Rosetta containing the pET22b-PTD-J-HSPAIA peptide II constructs grown in the presence of IPTG. First lane, standard molecular weight marker (bands corresponding to molecular mass of 15 kDa and 20 kDa are shown). Second lane (0), uninduced pET22b-PTD-J-HSPA1A peptide II; Third lane (1), induced pET22b-PTD-J-HSPAIA peptide II after 1 hour; Fourth lane (2), induced pET22b-PTD-J-HSPA1A peptide II after 2 hours; Fifth lane (3), induced pET22b-PTD-J-HSPA1A peptide II after 3 hours; Sixth lane (4), induced pET22b-PTD-J-HSPAIA peptide II after 4 hours; Seventh lane (5), induced pET22b-PTD-J-HSPAIA peptide II after 5 hours; Eighth lane (6), induced pET22b-PTD-J-HSPA1A peptide II after 6 hours; and Ninth lane (16), induced pET22b-PTD-J-HSPA1A peptide II after 16 hours.

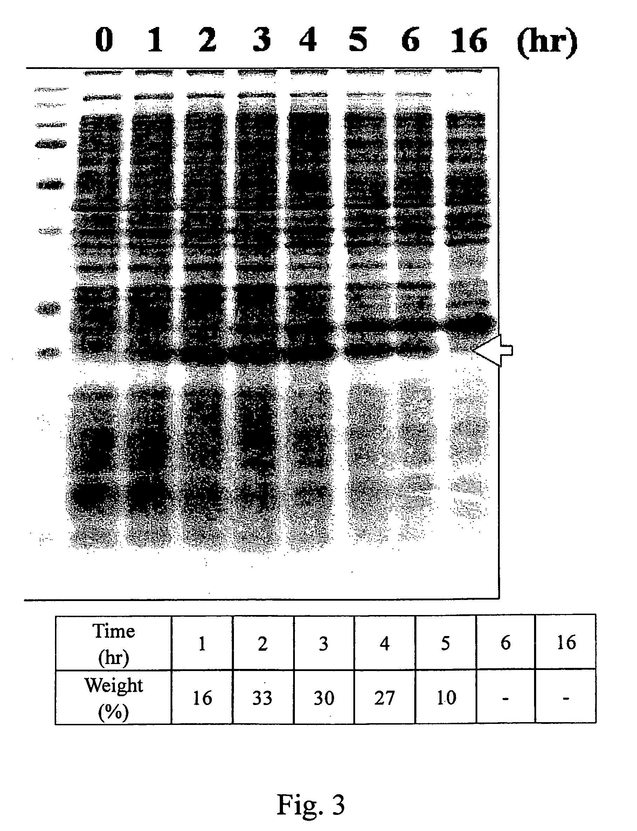

Fig. 3 shows a Coomassie® blue stained SDS-PAGE of whole cell lysates of *E*. *coli* Rosetta containing the pET22b-PTD-J-HSPAIA peptide III constructs grown in the presence of IPTG. First lane, standard molecular weight marker (bands corresponding to molecular mass of 15 kDa and 20 kDa are shown). Second lane (0), uninduced pET22b-PTD-J-HSPAIA peptide III; Third lane (1), induced pET22b-PTD-J-HSPA1A peptide III after 1 hour; Fourth lane (2), induced pET22b-PTD-J-HSPA1A peptide III after 2 hours; Fifth lane (3), induced pET22b-PTD-J-HSPAIA peptide III after 3 hours; Sixth lane (4), induced pET22b-PTD-J-HSPAIA peptide III after 4 hours; Seventh lane (5), induced pET22b-PTD-J-HSPAIA peptide III after 5 hours; Eighth lane (6), induced pET22b-PTD-J-HSPA1A peptide III after 6 hours; and Ninth lane (16), induced pET22b-PTD-J-HSPA1A peptide III after 16 hours.

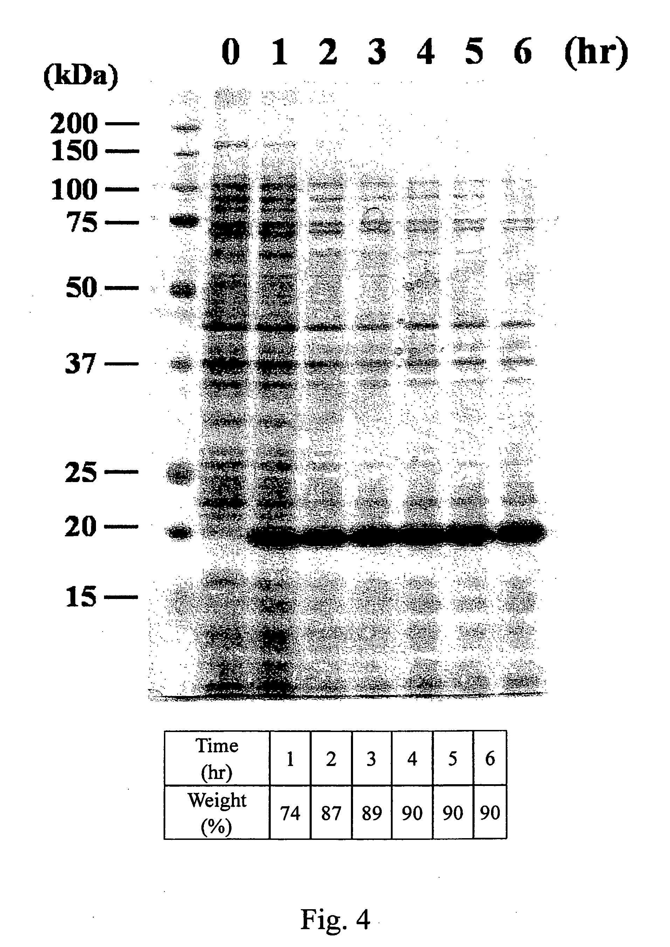

Fig. 4 shows a Coomassie® blue stained SDS-PAGE of whole cell lysates of *E. coli* Rosetta containing the pET22b-PTD-J-chicken IGF-I constructs grown in the presence of IPTG. First lane, standard molecular weight marker (bands corresponding to molecular mass of 15 kDa and 20 kDa are shown). Second lane (0), uninduced pET22b-PTD-J-chicken IGF-I; Third lane (1), induced pET22b-PTD-J-chicken IGF-I after 1 hour; Fourth lane (2), induced pET22b-PTD-J-chicken IGF-I after 2 hours; Fifth lane (3), induced pET22b-PTD-J-chicken IGF-I after 3 hours; Sixth lane (4), induced pET22b-PTD-J-chicken IGF-I after 4 hours; Seventh lane (5), induced pET22b-PTD-J-chicken IGF-I after 5 hours; and Eighth lane (6), induced pET22b-PTD-J-chicken IGF-I after 6 hours.

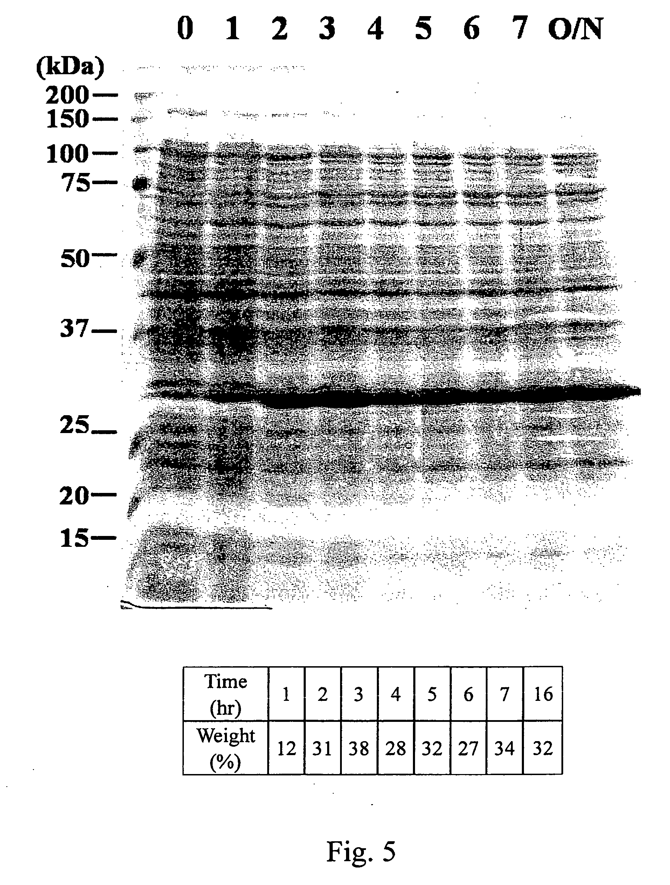

Fig. 5 shows a Coomassie® blue stained SDS-PAGE of whole cell lysates of *E. coli* Rosetta containing the pET22b-PTD-J-HA_RBD constructs grown in the presence of IPTG. First lane, standard molecular weight marker (bands corresponding to molecular mass of 25 kDa and 37 kDa are shown). Second lane (0), uninduced pET22b-PTD-J-HA_RBD; Third lane (1), induced pET22b-PTD-J-HA_RBD after 1 hour; Fourth lane (2), induced pET22b-PTD-J-HA_RBD after 2 hours; Fifth lane (3), induced pET22b-PTD-J-HA RBD after 3 hours; Sixth lane (4), induced pET22b-PTD-J-HA_RBD after 4 hours; Seventh lane (5), induced pET22b-PTD-J-HA_RBD after 5 hours; Eighth lane (6), induced pET22b-PTD-J-HA_RBD after 6 hours; Ninth lane (7), induced pET22b-PTD-J-HA_RBD after 7 hours; and Tenth lane (O/N), induced pET22b-PTD-J-HA_RBD after 16 hours.

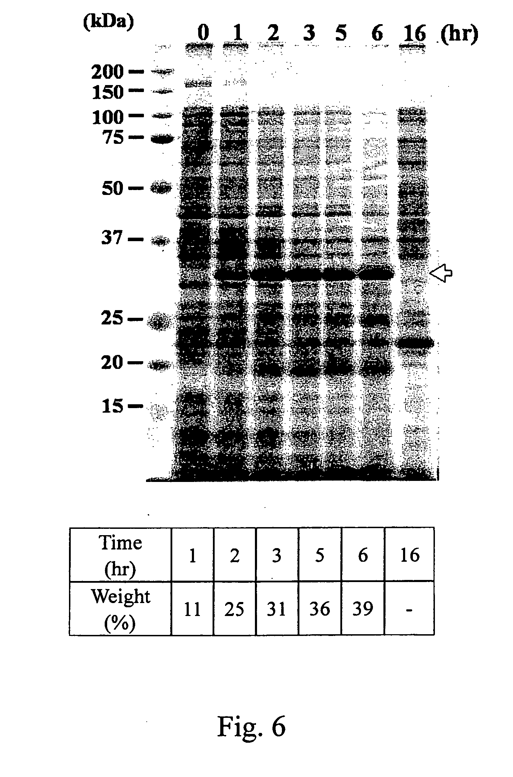

Fig. 6 shows a Coomassie® blue stained SDS-PAGE of whole cell lysates of *E*. *coli* Rosetta containing the pET22b-PTD-J-HCV core protein constructs grown in the presence of IPTG. First lane, standard molecular weight marker (bands corresponding to molecular mass of 25 kDa and 37 kDa are shown). Second lane (0), uninduced pET22b-PTD-J-HCV core protein; Third lane (1), induced pET22b-PTD-J-HCV core after 1 hour; Fourth lane (2), induced pET22b-PTD-J-HCV core protein after 2 hours; Fifth lane (3), induced pET22b-PTD-J-HCV core protein after 3 hours; Sixth lane (5), induced pET22b-PTD-J-HCV core protein after 5 hours; Seventh lane (6), induced pET22b-PTD-J-HCV core protein after 6 hours; and Eighth lane (16), induced pET22b-PTD-J-HCV core protein after 16 hours.

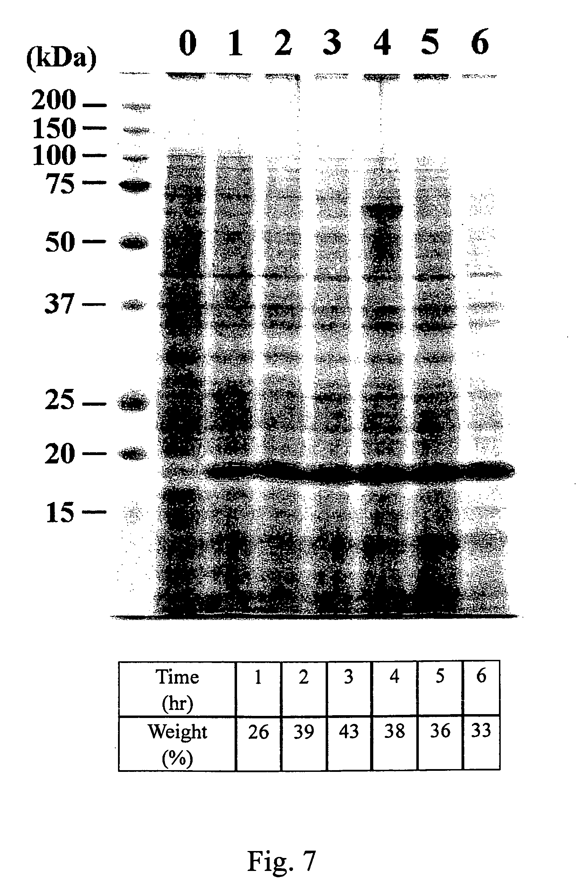

Fig. 7 shows a Coomassie® blue stained SDS-PAGE of whole cell lysates of *E*. *coli* Rosetta containing the pET22b-PTD-J-HpNC constructs grown in the presence of IPTG. First lane, standard molecular weight marker (bands corresponding to molecular mass of 15 kDa and 20 kDa are shown). Second lane (0), uninduced pET22b-PTD- J-HpNC; Third lane (1), induced pET22b-PTD- J-HpNC after 1 hour; Fourth lane (2), induced pET22b-PTD- J-HpNC after 2 hours; Fifth lane (3), induced pET22b-PTD- J-HpNC after 3 hours; Sixth lane (4), induced pET22b-PTD- J-HpNC after 4 hours; Seventh lane (5), induced pET22b-PTD- J-HpNC after 5 hours; and Eighth lane (6), induced pET22b-PTD- J-HpNC after 6 hours.

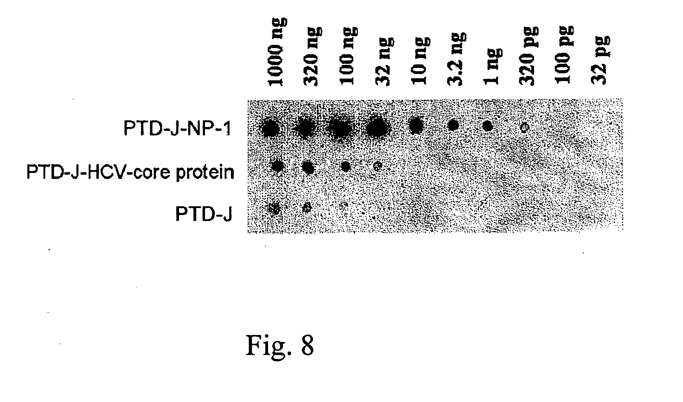

Fig. 8 is a dot blot for detecting the recombinant proteins PTD-J-neutrophile peptide-1 (NP-1), PTD-J-HCV-core protein and PTD-J at different concentrations in the serum.

### DETAILED DESCRIPTION OF THE INVENTION

The present invention is generally directed to an expression system, which is introduced into host cells to stably and efficiently produce a recombinant target protein with an enhanced yield and immunogenicity.

By the term "recombinant protein" as it is used herein is meant a recombinant molecule and usually a protein or peptide sequence covalently linked (i.e., fused) to a protein transduction domain and a J domain by recombinant, chemical or other suitable method. If desired, the recombinant molecule can be fused at one or several sites through a peptide linker sequence. That peptide sequence can include one or more sites for cleavage by a host cell induced protease.

A "polypeptide" refers to any polymer preferably consisting essentially of any of the 20 natural amino acids regardless of its size. Although the term "protein" is often used in reference to relatively large proteins, and "peptide" is often used in reference to small polypeptides, use of these terms in the field often overlaps. The term "polypeptide" refers generally to proteins, polypeptides, and peptides unless otherwise noted.

The term "immunogenicity" in accord with the invention refers to the ability of a recombinant protein, such as a recombinant antigenic protein to provoke an immune response.

In accordance with the invention, there is provided a method for expressing a recombinant target protein with an enhanced yield in a host cell, comprising constructing an expression vector comprising a nucleic acid segment consisting of a first nucleotide sequence encoding a protein transduction domain (PTD) of SEQ ID No:1, a second nucleotide sequence, linked in translation frame with the first nucleotide sequence, encoding a Hsp40 J-domain of SEQ ID NO:3 and a third nucleotide sequence, linked in translation frame with the second nucleotide sequence, encoding a target protein, wherein the nucleic acid segment is operatively linked to host specific transcription and translation regulatory elements for expressing the heterogeneous protein in the host cell. As an example, the nucleotide sequence encoding the PTD comprises a nucleotide sequence of SEQ ID NO: 2. And the nucleotide sequence of SEQ ID NO: 2 may be synthesized by using oligonucleotides of SEQ ID NOs: 5 and 6. The oligonucleotides of SEQ ID NO: 5 and 6 may be phosphorylated at their 5' ends. And the oligonucleotides may then be dephosphorylated by phosphatase once they are inserted in the vector. According to the invention, the Hsp40 J-domain of SEQ ID NO: 3 is encoded by a nucleotide sequence of SEQ ID NO: 4. And the nucleotide sequence of SEQ ID NO: 4 may be synthesized by using oligonucleotides of SEQ ID NOs: 7 to 16. And the target protein may include but not be limited to Hsp peptides, growth factors, virus receptor binding domains, and virus core proteins. In one example of the invention, the recombinant protein is an antigenic polypeptide which exhibit immunogenicity in the immunized hosts. In another example, the invention also provides a vaccine which comprises the recombinant protein produced with the above-described expression system.

The invention may provide nucleic acid sequences and particularly DNA sequences that encode the present recombinant proteins. In other examples, the DNA sequence may be carried by a vector suited for extrachromosomal replication such as a phage, virus, plasmid phagemid, cosmid, YAC or episome. In particular, a DNA vector that encodes a desired recombinant protein can be used to facilitate preparative methods described herein and to obtain significant quantities of the recombinant protein. The DNA sequence can be inserted into an appropriate vector, i.e., a vector that contains the necessary elements for the transcription and translation of the inserted protein-coding sequence. A variety of host-vector systems may be utilized to express the protein-coding sequence. These include mammalian cell systems infected with virus; insect cell systems infected with virus; microorganisms such as yeast containing yeast vectors, or bacteria transformed with bacteriophage DNA, plasmid DNA or cosmid DNA. Depending on the host-vector system utilized, and one of a number of suitable transcription and translation elements may be used. For example, the vector may contain promoters such as bacterial T7 promoter and inducible operator such as *lac* operator to regulate the transcription.

Other vectors and constructs include chromosomal, non chromosomal and synthetic DNA sequences, e.g., derivatives of SV40; bacterial plasmids; phage DNA; baculovirus; yeast plasmids; yeast artificial chromosomes (YACs); vectors derived from combination of plasmids and phage DNA; shuttle vectors derived from combinations of plasmids and viral DNA; viral DNA, such as vaccinia, adenovirus, fowl pox virus, and pseudorabies. However, any other vector may be used for preparation of a nucleic acid expression construct as long as it is replicable and viable in the host cell of interest. The nucleic acid sequence may be flanked by a number of restriction endonuclease sites for isolation and cloning into any desired vector. There are also protein identification or purification tags such as EE, 6XHis, HA or MYC added to facilitate subsequent purification of the recombinant protein. In addition, the nucleic acid expression construct may also contain a transcription terminator. For example, the vector may contain T7 terminator for terminating the transcription of the nucleic acid sequence.

In accordance with one specific example of the invention, recombinant proteins may be produced by using an expression vector which comprises a nucleotide sequence of SEQ ID No: 2, a nucleotide sequence encoding a Hsp40 J-domain of SEQ ID NO: 3, and a nucleotide sequence encoding a target protein. That is, the expression vector may include the nucleotide sequence of SEQ ID No: 2, a nucleotide sequence of SEQ ID NO: 4, and the nucleotide sequence encoding the target protein, as well as host specific transcription and translation regulatory elements operatively linked to the nucleotide sequences in the expression vector.

The vector carrying the recombinant molecules of the present invention may be efficiently transduced into target cells or groups of such cells. Transduction efficiency may be monitored and quantified if desired by one or a combination of different strategies.

For example, one approach involves an in vitro assay that measures uptake of the recombinant protein by the cell. The assay includes detectably-labeling the recombinant protein with e.g., a radioactive atom, fluorescent, phosphorescent, or luminescent tag (e.g. fluorescein, rhodamine, Cy3) and then measuring uptake of the labeled recombinant protein. Alternatively, the recombinant protein can be labeled with an enzyme capable of forming a detectable label such as horseradish peroxidase, β-galactosidase, chloramphenicol acetyl transferase or luciferase. Uptake can be measure by several conventional methods such as by quantifying labeled cells in a standard cell sorter (e.g., FACS), by fluorescence microscopy or by autoradiography.

As mentioned generally above, a host cell may be used for preparative purposes to propagate nucleic acid encoding a desired recombinant protein, Thus, the host cell may be a higher eukaryotic cell, such as mammalian cell, or a lower eukaryotic cell, such as a yeast cell, or the host cell can be a prokaryotic cell, such as a bacterial cell. Representative examples of appropriate host cells according to the present invention include, but need not be limited to, bacterial cells, such as *E*. *coli, Streptomyces, Salmonella typhimurium;* fungal cells, such as yeast; insect cells, such as *Drosophila* S2 and *Spodoptera* Sf9, animal cells, such as MDCK, Hep-2, CHO or COS; human cells, such as Jurkat or 293 cells; adenovirus; plant cells, or any other cells already adapted to in vitro propagation or so established de novo. The selection of an appropriate host cell is deemed to be within the scope of those skilled in the art from the teachings herein.

In addition, nucleic acid encoding a desired recombinant protein can be introduced into the host cells by standard techniques for transfecting cells. The term "transfecting" or 'transfection" is intended to encompass all conventional technique for introducing nucleic acid into host cells, including calcium phosphate co-precipitation, DEAE dextran-mediated transfection, lipofection, electroporation, microinjection, viral transduction and/or integration.

The present invention may further provide a production process for isolating a recombinant protein of interest. In the process, a host cell (e.g., a yeast, fungus, insect, bacterial or animal cell), into which has been introduced a nucleic acid encoding the protein of the interest operatively liked to a regulatory sequence, may be grown at production scale in a culture medium in the presence of the recombinant protein to stimulate transcription of the nucleotides sequence encoding the recombinant protein of interest. Subsequently, the recombinant protein of interest may be isolated from harvested host cells or from the culture medium. Standard protein purification techniques may be used to isolate the protein of interest from the medium or from the harvested cells. In particular, the purification techniques may be used to express and purify a desired fusion protein on a large-scale (i.e. in at least milligram quantities) from a variety of implementations including roller bottles, spinner flasks, tissue culture plates, bioreactor or a fermentor.

The recombinant proteins of the present invention may be separated and purified by appropriate combination of known techniques. These methods include, for example, methods utilizing solubility such as salt precipitation and solvent precipitation, methods utilizing the difference in molecular weight such as dialysis, ultra-filtration, gel-filtration, and SDS-polyacrylamide gel electrophoresis, methods utilizing a difference in electrical charge such as ionexchange column chromatography, methods utilizing specific affinity such as affinity chromatography, method utilizing a difference in hydrophobicity such as reverse-phase high performance liquid chromatography and methods utilizing a difference in isoelectric point, such as isoelectric focusing electrophoresis, metal affinity columns, such as Ni-NTA.

Therefore, in accordance with another example, the present invention may provide a method for producing recombinant target proteins with an enhanced yield and immunogenicity in a host cell. The method comprises:
constructing an expression vector comprising a nucleic acid segment consisting of a first nucleotide sequence encoding a protein transduction domain (PTD) of SEQ ID NO: 1, and a second nucleotide sequence, linked in translation frame with the first nucleotide sequence, encoding a Hsp40 J-domain of SEQ ID NO:3, and a third nucleotide sequence, linked in translation frame with the second nucleotide sequence, encoding a target protein, wherein the nucleic acid segment is operatively linked to host cell specific transcription and translation regulatory elements;
transforming the expression vector in the host cell; and
collecting and isolating the recombinant target protein from the host cell.

In accordance with one other example, the method further comprises a step of immunizing an organism with the recombinant protein. The antigenicity of the recombinant protein may be determined using labeled antibodies which bind to an epitope or epitopes of the recombinant protein blotted on a test membrane. Also, the immunogenicity of the recombinant protein may be compared with other polypeptides in antigen presentation assays *in vitro,* as well as in the induction of antibody responses and protection against subcutaneous challenge with the antigenic polypeptide *in vivo.*

The present invention also provide a chimeric protein comprising:
a. a first polypeptidyl fragment at the N-terminal end of the chimeric protein that consists of a protein transduction domain (PTD) of SEQ ID NO: 1;
b. a second polypeptidyl fragment at the C-terminal end of the first polypeptidyl fragment that consists of a Hsp40 J-domain of SEQ ID NO:3; and
c. a third polypeptidyl fragment at the C-terminal end of the second polypeptidyl fragment that contains a target protein or polypeptide.

The present invention also provides a chimeric gene comprising:
a. a first DNA sequence encoding a protein transduction domain (PTD) consisting of an amino acid sequence of SEQ ID NO:1; and
b. a second DNA sequence, linked in translation frame with the first DNA sequence, encoding a Hsp40 J-domain consisting of an amino acid sequence of SEQ ID NO:3.

The chimeric gene of the invention may further comprise a third DNA sequence, linked in translation frame with the second DNA sequence, encoding a target protein or polypeptide.

The antigenic polypeptide may include but need not be limited to Hsp peptides, growth factors, virus receptor binding domains, virus core proteins or combinations thereof. For example, other proteins or peptides capable of eliciting immune response to the host may also be encompassed in the invention.

The invention will now be described in further detail with reference to the following specific, non-limiting examples.

Example 1: Construction of pET22b-PTD-J-Ag expression vector

The pET22b plasmid (Novagen, Madison, Wis.) was utilized to create the pET22b-PTD-J vector which includes a nucleotide sequence encoding a protein transduction domain of SEQ ID No: 1, a nucleotide sequence encoding a Hsp40 J-domain of SEQ ID NO: 3, and a nucleotide sequence encoding a target protein, wherein the nucleic acid segment is operatively linked to host specific transcription and translation regulatory elements for expressing the recombinant target protein in the host cell. The oligonucleotides encoding optimized codons of PTD for *E*. *coli* were chemically synthesized using primers of SEQ ID NOs: 5 and 6 listed in Table I below. The 5'end of these two oliginucleotides were phosphorylated by polynucleotide kinase before annealing to double strand form in order to be inserted into the pET22b plasmid which was co-digested by *Nde*I and *Bam*HI and dephosphorylated by calf intestine alkaline phosphatase (CIAP) to create pET22b-PTD.

**Table I**

| | |
|---|---|
| PTD1 f | T ATG TAT GGT CGT AAG AAA CGT CGT CAG CGT CGT CGT GG (SEQ ID NO: 5) |
| PTD1 r | (p)GATCCC ACG ACG ACG CTG ACG ACG TTT CTT ACG ACC ATA CA (SEQ ID NO:6) |

The J-domain of Hsp40 gene was synthesized by assembly PCR using oligonucleotides of SEQ ID NOs: 7 to 16 shown in Table II. These oligonucleotides were designed for amplifying optimized codons for *E. coli.* The PCR product was cloned into pGEM-T Easy vector (Promega) for single colony selection and DNA sequencing. Plasmid with correct J-domain encoding DNA sequence was co-digested by *Bam*HI and *Eco*RI to remove the 0.2 kb inserted DNA fragment from pGEM-T Easy vector. This DNA fragment was then inserted into pET22b-PTD vector which was treated by *Bam*HI/*Eco*RI and CIAP to create pET22b-PTD-J expression vector.

**Table II**

| | |
|---|---|
| J1 nBF1 | tgg atc ctg ggt aaa gat tac tac cag act cac ggt ctc (SEQ ID NO: 7) |
| nF2 | tac tac cag act cac ggt ctc gct cgt ggt gca tct gat gat gaa atc (SEQ ID NO: 8) |
| F3 | ggt gca tct gat gat gaa atc aaa cgt gct tac cgt cgt cag gca ctg (SEQ ID NO:9) |
| F4 | gct tac cgt cgt cag gca ctg cgt tac cat cca gac aaa aac aaa gaa (SEQ ID NO: 10) |
| F5 | tac cat cca gac aaa aac aaa gaa ccg ggt gca gaa gag aaa ttc (SEQ ID NO: 11) |
| F6 | ccg ggt gca gaa gag aaa ttc aaa gag atc gca gaa gca tac gac gtt (SEQ ID NO: 12) |
| *Eco*RI R1 | cga att cgc acc acc aga acc acc ttt cag acc ttc (SEQ ID NO:13) |
| R2 | aga acc acc ttt cag acc ttc ttc acc gta acg gtc gaa gat ttc acg (SEQ ID NO: 14) |
| R3 | gta acg gtc gaa gat ttc acg ttt acg tgg atc gct cag aac gtc gta (SEQ ID NO: 15) |
| R4 | tgg atc gct cag aac gtc gta tgc ttc tgc gat ctc (SEQ ID NO: 16) |

One of more recombinant polypeptide or antigenic (Ag) polypeptide to be expressed by pET22b-PTD-J vector was usually transformed to DNA with optimized *E*. *coli* codons. The one or more recombinant polypeptide encoding regions were amplified by PCR too. These polypeptide encoding DNAs in the following examples were flanked by *Eco*RI and *Xho*I site in order to be inserted into the pET22b-PTD-J vector conveniently. As a result, a pET22b-PTD-J-Ag expression vector was constructed.

Assembly PCR

Oligonucleotides sets were utilized to synthesize codon optimized cDNA. 0.5 µM of F1 and R1 as well as 0.05 µM of F2, F3, R3, R2 and so on were adjusted in PCR reaction mixture. The reaction conditions were 94°C for 2 min followed by 20 cycles of 94°C for 20 sec / 40°C for 40 sec / 72°C for 20 sec and extension at 72°C for 5 min. The PCR products were cloned into pGEM-T Easy for plasmid DNA isolation and DNA sequencing.

Example 2: expression of PTD-J-Ag recombinant proteins

The pET22b-PTD-J-Ag vectors were transformed into *E*. *coli* Rosetta (Novagen) competent cells. The *E*. *coli* colonies resistant to ampicillin and chloramphenicol were cultured and amplified in TYD medium (10 g trypton / 20 g yeast extract / 5 g NaCl / 2 g Dextrose per liter, pH 7.2) to OD₆₀₀ = 0.3 before induction with 1 mM IPTG. The cell samples were collected at time schedule listed.

Total protein samples according to 0.3 OD₆₀₀ unit of *E*. *coli* were lyzed in 30 µL of 2× SDS-PAGE loading buffer. After treated by boiling water bath for 5 min, these protein samples were separated by 12.5% or 15% SDS-PAGE. The protein bands were visualized by performing Coomassie brilliant blue R250 staining on the SDS-PAGE gel.

Example 3: Immunization

Mice were immunized subcutaneously with 50 µg polypeptides which were emulsified with TiterMAX Gold adjuvant. Boosts were carried out every second week. The sera of the mice were collected to raise antibodies against the polypeptides.

Dot blotting

Serially diluted recombinant protein or synthetic polypeptide were spotted onto PVDF membranes. The membranes were blocked by 5% skim milk in TBSN (25 mM Tris-HCl, pH 7.4 / 150 mM NaCl / 0.02% Tween 20) for 1 hour. After 4 washes with TBSN, the membranes were soaked in mouse anti-serum which was diluted for 1000 folds for overnight incubation. Unbound primary antibodies (Ab) were removed by 4 washes with TBSN then HRP-conjugated goat antimouse secondary Ab (2000 folds dilution) was applied for 4 hours. The labeled proteins on washed membrane were performed by chemo-luminescence kit (Pierce) as described by manufacture's instruction.

Example 4: Expression of human HSPA1A Peptide I

The oligonucleotides used for assembly PCR were listed in Table III. They were utilized to synthesize codon optimized cDNA of SEQ ID NO: 17 which encodes the HAPA1A peptide I of SEQ ID NO: 18 (SSSTQASLEI DSLFEGIDFY TSITRARFEE LCSDLFRSTL EPVEKALRDA KLDKAQI). The codon optimized cDNA sequence was inserted in the expression vector pET22b-PTD-J constructed according to the Example 1.

**Table III**

| | |
|---|---|
| RI-I-f1 | |
| I-f2 | |
| I-f3 | |
| I-r3 | |
| I-r2 | |
| Xho-I-r1 | |

The expression vector having a nucleic acid segment that encoded recombinant PTD-J-HSPA1A peptide I was introduced into *E*. *coli* as described in the example 2. And the recombinant PTD-J-HSPA1A peptide I expression was quantitated and represented in terms of weight percentage of the recombinant PTD-J-HSPA1A peptide I over total protein. Referring to Fig. 1, the weight percentage of the recombinant PTD-J-HSPA1A peptide I ranges from 65% to 92% for the first 6 hours. And the weight percentage of the recombinant PTD-J-HSPA1A peptide I was 77% after 16 hours.

Example 5: Expression of human HSPA1A Peptide II

The oligonucleotides used for assembly PCR were listed in Table IV. They were utilized to synthesize codon optimized cDNA of SEQ ID NO: 25 which encodes the HAPA1A peptide II of SEQ ID NO: 26 (NVTATDKSTG KANKITITND KGRLSKEEIE RMVQEAEKYK AEDEVQRERV SAKNALESYA F). The codon optimized cDNA sequence was inserted in the expression vector constructed according to the Example 1.

**Table IV**

| | |
|---|---|
| RI-II-f1 | |
| II-f2 | |
| II-f3 | |
| II-f4 | |
| II-r3 | |
| II-r2 | |
| Xho-II-r1 | |

The expression vector having a nucleic acid segment that encoded recombinant PTD-J-HSPA1A peptide II was introduced into *E*. *coli* as described in the example 2. And the recombinant PTD-J-HSPA1A peptide II expression was quantitated and represented in terms of weight percentage of the recombinant PTD-J-HSPA1A peptide II over total protein. Referring to Fig. 2, the weight percentage of the recombinant PTD-J-HSPA1A peptide II ranges from 27% to 54% for the first 6 hours. And the weight percentage of the recombinant PTD-J-HSPA1A peptide II was 10% after 16 hours.

Example 6: Expression of human HSPA1A Peptide III

The oligonucleotides used for assembly PCR were listed in Table V. They were utilized to synthesize codon optimized cDNA of SEQ ID NO: 34 which encodes the HSPA1A peptide III of SEQ ID NO: 35 (EDEGLKGKIS EADKKKVLDK CQEVISWLDA NTLAEKDEFE HKRKELEQVC NPIISGLYQG A). The codon optimized cDNA sequence was inserted in the expression vector constructed according to the Example 1.

**Table V**

| | |
|---|---|
| RI-III-f1 | |
| III-f2 | |
| III-f3 | |
| III-f4 | |
| III-r3 | |
| III-r2 | |
| Xho-III-r1 | |

The expression vector having a nucleic acid segment that encoded recombinant PTD-J-HSPA1A peptide III was introduced into *E*. *coli* as described in the example 2. And the recombinant PTD-J-HSPA1A peptide III expression was quantitated and represented in terms of weight percentage of the recombinant PTD-J-HSPA1A peptide II over total protein. Referring to Fig. 3, the weight percentage of the recombinant PTD-J-HSPA1A peptide I ranges from 10% to 33% for the first 5 hours.

Example 7: Expression of chicken IGF-I

The oligonucleotides used for assembly PCR were listed in Table VI. They were utilized to synthesize codon optimized cDNA of SEQ ID NO: 43 which encodes the chicken IGF-I of SEQ ID NO: 44 (GPETLCGAEL VDALQFVCGD RGFYFSKPTG YGSSSAALHH KGIVDECCFQ SCDLRRLEMY CAPIKPPKSA). The codon optimized cDNA sequence was inserted in the expression vector constructed according to the Example 1.

**Table VI**

| | |
|---|---|
| RI-F1 new | |
| F2 | |
| F3 | |
| F4 | |
| F5 | |
| R2 | |
| Xho-R1 | |

The expression vector having a nucleic acid segment that encoded recombinant PTD-J-chicken IGF-I was introduced into *E*. *coli* as described in the example 2. And the recombinant PTD-J-chicken IGF-I expression was quantitated and represented in terms of weight percentage of the recombinant PTD-J-chicken IGF-I over total protein. Referring to Fig. 4, the weight percentage of the recombinant PTD-J-chicken IGF-I ranges from 74% to 90% for the first 6 hours.

Example 8: Expression of avian Influenza virus HA (H5) receptor binding domain (RBD)

The primer pair used for PCR was listed in Table VII. They were utilized to synthesize native viral cDNA of SEQ ID NO: 52 which encode the HA_RBD of SEQ ID NO: 53. The cDNA sequence was inserted in the expression vector constructed according to the Example 1.

**Table VII**

| | |
|---|---|
| RI HA-RBD f | TG AAT TCG AAA CAC CTA TTG AGC AGA ATA AAC (SEQ ID NO: 54) |
| Xho HA-RBD r | TCTCGAG AAT TGT TGA GTC CCC TTT CTT GAC (SEQ ID NO: 55) |

The expression vector having a nucleic acid segment that encoded recombinant PTD-J-HA_RBD was introduced into *E*. *coli* as described in the example 2. And the recombinant PTD-J-HA_RBD expression was quantitated and represented in terms of weight percentage of the recombinant PTD-J-HA_RBD over total protein. Referring to Fig. 5, the weight percentage of the recombinant PTD-J-HA_RBD ranges from 12% to 38% for the first 7 hours. And the weight percentage of the recombinant PTD-J-HA_RBD was 32% after 16 hours.

Example 9: Expression of Hepatitis C virus (HCV) core protein

The primer pair used for PCR was listed in Table VIII. They were utilized to synthesize native viral cDNA of SEQ ID NO: 56 which encode the HCV core protein of SEQ ID NO: 57. The cDNA sequence was inserted in the expression vector constructed according to the Example 1.

**Table VIII**

| | |
|---|---|
| RI-Core f | TG AAT TCG ATG AGC ACA AAT CCT AAA CCT C (SEQ ID NO: 58) |
| Xho-Core r | TCTCGAG AGC AGA GAC CGG AAC GGT GAT (SEQ ID NO: 59) |

The expression vector having a nucleic acid segment that encoded recombinant PTD-J-HCV core protein was introduced into *E*. *coli* as described in Example 2. And the recombinant PTD-J-HCV core protein expression was quantitated and represented in terms of weight percentage of the recombinant PTD-J-HCV core protein over total protein. Referring to Fig. 6, the weight percentage of the recombinant PTD-J-HCV core protein ranges from 11 % to 39% for the first 6 hours.

Example 10: Increased HpNC immunogenicity

The primer pair used for PCR was listed in Table IX. They were utilized to synthesize the codon-optimized cDNA sequence coding for the human haptoglobin fused peptide HpNC of SEQ ID NO: 60 which encodes the HpNC protein of SEQ ID NO:61. The fused peptide HpNC of human haptoglobin α-chain was composed of the N-terminal DSGNDVTDIADDG peptide, which is present in the mature secreted α1 and α2 peptide of haptoglobin, a linker GSGG tetra-peptide, and the C-terminal RHYEGSTVPEKKTPKS peptide, which is only present in pre-mature haptoglobin α-chain. The codon-optimized cDNA sequence was inserted into the expression vector pET22b-PTD-J constructed according to Example 1.

**Table IX**

| | |
|---|---|
| RI-F1 | AGAATTCTGATAGCGGCAACGATGTGACCGATATTGCGGATGATGGTGG (SEQ ID NO:62) |
| F2 | TGACCGATATTGCGGATGATGGTGGTAGTGGTGGTCGTCATTAT (SEQ ID NO:63) |
| F3 | ATGGTGGTAGTGGTGGTCGTCATTATGAAGGTAGCACCGTT (SEQ ID NO: 64) |
| R2 | GGTTTTCTTCTCCGGAACGGTGCTACCTTCATAATGACGACCACCAC (SEQ ID NO:65) |
| Xho-R1 | TCTCGAGACCA CCGCTCTTTGGGG TTTTCTTCTCCGGAACGGTGCTA (SEQ ID NO:66) |

The expression vector having a nucleic acid segment that encoded recombinant pET22b-PTD-J-HpNC was introduced into *E*. *coli* as described in Example 2. And the recombinant PTD- J-HpNC protein expression was quantitated and represented in terms of weight percentage of the recombinant PTD- J-HpNC protein over total protein. Referring to Fig. 7, the weight percentage of the recombinant PTD- J-HpNC protein ranges from 26% to 33% for the first 6 hours.

From the results of the above examples, it was evident that the weight percentage of the recombinant protein produced by using the pET22b-PTD-J expression vector with the optimized codons was demonstrated to be around 90% relative to total protein. On the other hand, if native cDNA was used, the weight percentage of recombinant protein within total proteins could be around 40%. Therefore, the expression vector of the invention allows production of the recombinant proteins with enhanced yield.

Example 11: Increase Rabbit Neutrophile peptide-1 (NP-1) immunogenicity

The codon optimized cDNA sequence encoding NP-1 (SEQ ID NO: 67) was synthesized and inserted in the expression vector pET22b-PTD-J constructed according to the method described in Example 1. The expression vector having a nucleic acid segment that encoded recombinant pET22b-PTD-J-NP-1 was introduced into *E*. *coli* as described in Example 2. And the recombinant NP-1 (SEQ ID NO: 68) was collected and isolated from the *E*. *coli.*

The mice were immunized by injecting subcutaneously with the recombinant NP-1 according to the Example 3 to raise the antibodies against the recombinant NP-1. Serum was then collected from the mice. The antigenicity of the NP-1 was determined using the dot blotting assay described in Example 3.

A highly tittered serum was raised when the recombinant NP-1 was utilized as antigen. The serum could detect 320 pg of antigen but did not cross-react to PTD-J-HCV-core recombinant protein, indicating that the serum was strictly against the NP-1 region both strongly and specifically as shown in Fig. 8.

## Claims

1. A method for producing a recombinant target protein with an enhanced yield in a host cell comprising:
constructing an expression vector comprising a nucleic acid segment consisting of a first nucleotide sequence encoding a protein transduction domain (PTD) of SEQ ID NO: 1, a second nucleotide sequence, linked in translation frame with the first nucleotide sequence, encoding a heat shock protein 40 (Hsp40) J-domain of SEQ ID NO:3, and a third nucleotide sequence, linked in translation frame with the second nucleotide sequence, encoding a target protein, wherein the nucleic acid segment is operatively linked to host cell specific transcription and translation regulatory elements;
transforming the expression vector in the host cell; and
collecting and isolating the recombinant target protein from the host cell.

2. The method according to claim 1, wherein the recombinant target protein produced has enhanced immunogenicity.

3. A chimeric protein comprising:
a. a first polypeptidyl fragment at the N terminal end of the chimeric protein consisting of a protein transduction domain (PTD) of SEQ ID NO:1;
b. a second polypeptidyl fragment at the C terminal end of the first polypeptidyl fragment consisting of a Hsp40 J-domain of SEQ ID NO:3; and
c. a third polypeptidyl fragment that contains a target protein or polypeptide.

4. A chimeric gene comprising:
a. a first DNA sequence encoding a protein transduction domain (PTD) consisting of an amino acid sequence of SEQ ID NO:1; and
b. a second DNA sequence, linked in translation frame with the first DNA sequence encoding a Hsp40 J-domain consisting of an amino acid sequence of SEQ ID NO:3.

5. A chimeric gene according to claim 4, further comprising a third DNA sequence linked in translation frame with the second DNA sequence encoding a target protein or polypeptide.

6. A host comprising an expression vector comprising a chimeric gene according to claim 4 or 5.

## Patentansprüche

1. Ein Verfahren zur Herstellung eines rekombinanten Zielproteins mit einer erhöhten Ausbeute in einer Wirtszelle umfassend:
Konstruieren eines Expressionsvektors, der ein Nukleinsäuresegment umfasst, das aus einer ersten Nukleotidsequenz, die eine Protein-Transduktions-Domäne (PTD) gemäß SEQ ID NO:1 kodiert, einer zweiten Nukleotidsequenz, die in einem Translationsrahmen mit der ersten Nukleotidsequenz verknüpft ist und eine Hitze-Schock-Protein 40 (Hsp40) J-Domäne gemäß SEQ ID NO:3 kodiert und einer dritten Nukleotidsequenz, die in einem Translationsrahmen mit der zweiten Nukleotidsequenz verknüpft ist und ein Zielprotein kodiert, besteht, wobei das Nukleinsäuresegment funktional mit Wirtszell-spezifischen Transkriptions- und Translations-regulierenden Elementen verknüpft ist;
Transformieren der Wirtszelle mit dem Expressionsvektor; und
Sammeln und Isolieren des rekombinanten Zielproteins aus der Wirtszelle.

2. Das Verfahren nach Anspruch 1, wobei das produzierte rekombinante Zielprotein eine erhöhte Immunogenität besitzt.

3. Ein chimäres Protein umfassend:
a. ein erstes Polypeptidylfragment am N-terminalen Ende des chimären Proteins, das aus einer Protein-Transduktions-Domäne (PTD) gemäß SEQ ID NO:1 besteht;
b. ein zweites Polypeptidylfragment am C-terminalen Ende des ersten Polypeptidylfragments, das aus einer Hsp40 J-Domäne gemäß SEQ ID NO:3 besteht; und
c. ein drittes Polypeptidylfragment, das ein Zielprotein oder Polypeptid beinhaltet.

4. Ein chimäres Gen umfassend:
a. eine erste DNA-Sequenz, die eine Protein-Transduktions-Domäne (PTD) kodiert, die aus einer Aminosäuresequenz gemäß SEQ ID NO:1 besteht; und
b. eine zweite DNA-Sequenz, die in einem Translationsrahmen mit der ersten DNA-Sequenz verknüpft ist und eine Hsp40 J-Domäne kodiert, die aus einer Aminosäuresequenz gemäß SEQ ID NO:3 besteht.

5. Ein chimäres Gen nach Anspruch 4, ferner umfassend eine dritte DNA-Sequenz, die in einem Translationsrahmen mit der zweiten DNA-Sequenz verknüpft ist und ein Zielprotein oder Polypeptid kodiert.

6. Eine Wirtszelle umfassend einen Expressionsvektor, der ein chimäres Gene nach Anspruch 4 oder 5 umfasst.

## Revendications

1. Procédé de production d'une protéine cible recombinante avec un rendement amplifié dans une cellule hôte comprenant :
la construction d'un vecteur d'expression comprenant un segment d'acide nucléique constitué par une première séquence nucléotidique codant pour un domaine de transduction de protéine (DTP) de SEQ ID NO : 1, une deuxième séquence nucléotidique, liée dans le cadre de la traduction avec la première séquence nucléotidique, codant pour le domaine J d'une protéine de choc thermique 40 (Hsp40) de SEQ ID NO : 3, et une troisième séquence nucléotidique, liée dans le cadre de la traduction avec la deuxième séquence nucléotidique, codant pour une protéine cible, dans lequel le segment d'acide nucléique est lié de manière fonctionnelle à des éléments régulateurs de la transcription et de la traduction spécifiques de la cellule hôte ;
la transformation du vecteur d'expression dans la cellule hôte ; et
le recueil et l'isolement de la protéine cible recombinante à partir de la cellule hôte.

2. Procédé selon la revendication 1, dans lequel la protéine cible recombinante produite présente une immunogénicité amplifiée.

3. Protéine chimérique comprenant :
a. un premier fragment polypeptidyle à l'extrémité N-terminale de la protéine chimérique constitué par un domaine de transduction de protéine (DTP) de SEQ ID NO : 1 ;
b. un deuxième fragment polypeptidyle à l'extrémité C-terminale du premier fragment polypeptidyle constitué par un domaine J de Hsp40 de SEQ ID NO : 3 ; et
c. un troisième fragment polypeptidyle qui contient une protéine ou un polypeptide cible.

4. Gène chimérique comprenant :
a. une première séquence d'ADN codant pour un domaine de transduction de protéine (DTP) constitué par une séquence d'acides aminés de SEQ ID NO : 1 ; et
b. une deuxième séquence d'ADN, liée dans le cadre de la traduction avec la première séquence d'ADN codant pour un domaine J de Hsp40 constitué par une séquence d'acides aminés de SEQ ID NO : 3.

5. Gène chimérique selon la revendication 4, comprenant en outre une troisième séquence d'ADN liée dans le cadre de la traduction avec la deuxième séquence d'ADN codant pour une protéine ou un polypeptide cible.

6. Hôte comprenant un vecteur d'expression comprenant un gène chimérique selon la revendication 4 ou 5.
